# EUROPÄISCHE PATENTSCHRIFT

(11) **EP 0 377 437 B1**
(45) Veröffentlichungstag und Bekanntmachung des Hinweises auf die Patenterteilung: **07.12.1994**
(21) Anmeldenummer: 90100029.9
(22) Anmeldetag: 02.01.1990
(51) Int. Cl.: C07D 239/50, C07D 239/48

(54) **Verfahren zur Herstellung von 2,4-Diamino-6-piperidinyl-pyrimidin-3-N-oxid**
Process for the preparation of 2,4-diamino-6-piperidinyl-pyrimidine-3-N-oxide
Procédé pour la préparation de 2,4-diamino-pipéridinyl-pyrimidine-3-N-oxyde

(30) Priorität: 04.01.1989 CH 20/89
(43) Veröffentlichungstag der Anmeldung: 11.07.1990
(73) Patentinhaber: LONZA AG, CH-3945 Gampel/Wallis (CH)
(72) Erfinder: Mills, Lester, Dr., Naters (Wallis) (CH); Mettler, Hans Peter, Dr., Brig-Glis (Wallis) (CH); Previdoli, Felix, Dr., Brig (Wallis) (CH); Moulin, François, Dr., Neuchâtel (CH)
(74) Vertreter: Weinhold, Peter, Dr.

(56) Entgegenhaltungen:
- EP-A- 0 115 325
- EP-A- 0 252 515
- EP-A- 0 254 158
- GB-A- 1 450 010
- GB-A- 2 032 434
- JOURNAL OF ORGANIC CHEMISTRY, Band 40, Nr 22, 1975, Seiten 3304-3306, Easton, PA, US; J.M. McCall et al.: "A New Approach to Triaminopyrimidine N-Oxides"

## Beschreibung

Die vorliegende Erfindung betrifft ein Verfahren zur Herstellung von 2,4-Diamino-6-piperidinylpyrimidin-3-N-oxid (Minoxidil).
Minoxidil zeichnet sich durch eine hervorragende blutdrucksenkende Wirkung aus (Drugs 22 [1981], 257) und ist in zahlreichen Ländern als blutdrucksenkendes Mittel bekannt. In letzter Zeit gerät die Verwendung der Verbindung als Heilkosmetikum immer mehr in den Vordergrund, da sie in verdünnter Lösung äusserlich angewandt das Haarwachstum wirksam anregt (Pharm. Ind. 46 [1984], 937 und Pharm. Ind. 47 [1985], 506). Im Fachschrifttum hat die Bezeichnung Minoxidil - da die Verbindung in 2 tautomeren Formen vorkommen kann - 2fache Bedeutung. In den Chemical Abstracts wurde die Verbindung vor 1972 unter dem Namen 6-Amino-1,2-dihydro-1-hydroxy-2-imino-4-piperidino-pyrimidin erwähnt, während sie danach unter der Bezeichnung 6-(1-Piperidinyl)-2,4-pyrimidindiamin-3-oxid referiert wird.
Für die Herstellung von Minoxidil sind mehrere Verfahren bekannt. Die einen Verfahren gehen von 2,6-Diamino-4-chlorpyrimidin aus. Durch Oxidation mit z.B. Chlorperbenzoesäure oder Wasserstoffperoxid werden die entsprechenden N-Oxide hergestellt, die dann weiter umgesetzt werden (EP-A 0 254 158). Ein weiteres bekanntes Verfahren geht von Acyl- und/oder Acyloxygruppen enthaltenden 2-Imino-pyrimidinderivaten, z.B. von 6-Amino-1,2-dihydro-1-acetoxy-2-imino-4-chlorpyrimidin aus. Durch Umsetzung mit Piperidin wird die entsprechende Piperidino-Verbindung hergestellt, die anschliessend hydrolysiert wird (EP-A 0 252 515).
Diese bekannten Verfahren sind entweder technisch sehr aufwendig oder die Ausbeuten lassen zu wünschen übrig.
Ein anderes bekanntes Verfahren wird in J.Org.Chem. 40 [1975], 3304 beschrieben. Das dort verwendete Schlüsselprodukt ist Cyanacetylpiperidinsäureamid, dessen Carboxylgruppe erst reaktionsfähig gemacht werden muss. Für dieses Verfahren sind nur schwer zugängliche und mit hohem Aufwand verbundene Stoffe erforderlich.

Der Erfindung liegt daher die Aufgabe zugrunde, unter Behebung der Nachteile der bekannten Verfahren, ein technisch bzw. industriell und wirtschaftlich durchführbares Verfahren zur Herstellung von Minoxidil, in guter Ausbeute und in grosser Reinheit, zu schaffen. Dies wurde überraschenderweise durch die Erfindung gemäss den Patentansprüchen erreicht.

Das Zwischenprodukt 2,4-Diamino-6-hydroxypyrimidin-3-N-oxid ist neu und kann als Hydrat oder in Form seiner Salze auftreten.

Minoxidil bzw. die im erfindungsgemässen Verfahren auftretenden Verbindungen unterliegen der Tautomerie, d.h. 2,4-Diamino-6-piperidinyl-pyrimidin-3-N-oxid steht in einem tautomeren Gleichgewicht zu 6-Amino-1,2-dihydro-1-hydroxy-2-imino-4-piperidinylpyrimidin. Der Einfachheit halber wird in den folgenden Ausführungen nur die N-oxid-form der Verbindungen erwähnt.
Eine bevorzugte Ausführungsform der Erfindung besteht darin, dass die in situ-Bildung des Hydroxyguanidins durch Umsatz von Hydroxylamin, vorzugsweise in Form des Hydrochlorids, mit Cyanamid in Methanol in Gegenwart von Na-methylat erfolgt. Zweckmässig verwendet man die Edukte sowie das Na-methylat in molarem Verhältnis.
Die Verweilzeit beträgt zweckmässig 2 bis 4 Stunden und die bevorzugte Temperatur liegt bei 0 bis 20°C.
Danach wird das gebildete Natriumchlorid zweckmässigerweise abfiltriert.
Ausser Methanol können aber auch andere niedrige Alkohole, wie Ethanol, n-Propanol und andere Alkalialkoholate, wie Ethylat, n-Propylat angewendet werden.
Eine weitere Möglichkeit zum Hydroxyguanidin zu gelangen, besteht darin, dass man in einer ersten Stufe eine wässrige Cyanamidlösung mit einer wässrigen Halogenwasserstofflösung zum entsprechenden Halogenformamidiniumhalogenid umsetzt, diesen isoliert und - wie vorstehend beschrieben - dieses Zwischenprodukt mit Hydroxylamin in Gegenwart eines Alkalialkoholats in einen niederen Alkohol zum Hydroxyguanidin umsetzt. Die Methode, das Halogenformamidiniumhalogenid herzustellen, ist aus der DE-OS 1 915 668 bekannt.
Vorzugsweise wird durch Umsetzung einer wässrigen Cyanamidlösung mit einer wässrigen Salzsäure das Chlorformamidiniumchlorid hergestellt.
Anschliessend wird der Cyanessigester, vorzugsweise der Methylester, zweckmässig in molarem Verhältnis und weiteres Natriummethylat, gelöst in Methanol, zugesetzt.
Die Temperatur wird während der Zugabe zweckmässig bei 0 bis 5°C gehalten.
Anschliessend wird die Reaktionsmischung 1 bis 2 Stunden bei Temperaturen von 10 bis 20°C gehalten und danach auf Rückflusstemperatur gebracht. Die Verweilzeit bei Rückfluss liegt zweckmässig bei 4 bis 6 Stunden.
Das gebildete 2,4-Diamino-6-hydroxypyrimidin-3-N-oxid wird vorzugsweise wasserfrei oder als Hydrat isoliert und anschliessend der Chlorierungsstufe zugeführt. Das molare Verhältnis 2,4-Diamino-6-hydroxypyrimidin-3-N-oxid zu POCl₃ und N,N-Dimethylanilin, als bevorzugter Katalysator, liegt bei 1 zu 5 zu 1 bis 1 zu 10 zu 3.
Neben dem bevorzugten N,N-Dimethylanilin können auch andere Katalysatoren, wie N,N-Diethylaniline, Triethylamin, Tributylamin angewendet werden.
Die Reaktionstemperatur liegt bei 70 bis 100°C, vorzugsweise bei 80 bis 85°C. Die Reaktionsdauer liegt bei 10 bis 80 Stunden, vorzugsweise bei 10 bis 20 Stunden.
Zweckmässigerweise wird das gebildete 2,4-Diamino-6-chlorpyrimidin-N-oxid als freie Verbindung oder als z.B. Hydrochlorid isoliert und anschliessend der Umsetzung mit Piperidin zugeführt. Dabei werden pro Mol Chlorierungsprodukt zweckmässig 15 bis 25 Mol Piperidin angewendet. Die bevorzugte Temperatur liegt bei 70 bis 104°C, die Reaktionsdauer bei 2 bis 5 Stunden.

### Beispiel 1

### a) Herstellung von 2,4-Diamino-6-hydroxypyrimidin-N-oxid Hydrat

6,95 g Hydroxylaminhydrochlorid (0,1 Mol), 4,2 g Cyanamid (0,1 Mol) und 15 g Methanol wurden bei 0°C zusammengegeben und während 3 Stunden wurden 5,4 g Natriummethylat (30% in Methanol, 0,1 Mol) zudosiert. Der Natriumchloridniederschlag wurde abfiltriert und zum Filtrat 9,9 g Cyanessigsäuremethylester (0,1 Mol) zugegeben. Bei 0 bis 5°C wurden weitere 5,4 g Natriummethylat (30% in Methanol, 0,1 Mol) zudosiert. Das Reaktionsgemisch wurde während 1 Stunde bei 15°C und anschliessend während 4 Stunden bei Rückflusstemperatur gerührt. Das Methanol wurde unter Vakuum entfernt und 60 g Wasser zugegeben. Das Gemisch wurde bei 40°C mit 20%iger Salzsäurelösung auf pH 4,5 gestellt und auf Raumtemperatur abgekühlt. Der Festkörper wurde abfiltriert, mit Wasser gewaschen und bei 50°C/20 Torr getrocknet. Man erhielt 7,02 g braunes Pulver mit einem Gehalt von 86,5% (Rohprodukt). Die Ausbeute betrug 42,7% bezogen auf Cyanamid. Smp. über 300°C

| Elementaranalyse für das Hydrat | | | |
|---|---|---|---|
| ber. | C 30,0% | H 5,0% | N 35,0% |
| gef. | C 30,1% | H 5,0% | N 34,4% |

- ¹H-NMR (DMSO-d6): 5,15 (s, 1H)
7,15 (bs, 2H)
7,4 (bs, 2H)
OH-Gruppe kommt mit Wasserpeak zusammen
- ¹³C-NMR (DMSO-d6): 80,0 - 155,8 - 158,0 - 164,5

### b) Herstellung von 6-Chlor-2,4-diaminopyridin-3-N-oxid

5,0 g 2,4-Diamino-6-hydroxypyrimidin-3-N-oxid Hydrat (0,035 Mol), 40 g Phosphoroxychlorid (0,26 Mol) und 4,25 g N,N-Dimethylanilin (0,0352 Mol) wurden zusammengegeben und bei 82°C während 60 Stunden gerührt. Das überschüssige Phosphoroxychlorid wurde unter Vakuum abdestilliert. Zum Rückstand wurden vorsichtig 100 g Wasser zugegeben. Nach 1 Stunde bei Raumtemperatur wurde der pH mit 25%iger Natronlauge auf 9 gestellt, wobei das Reaktionsgemisch gekühlt wurde. Das Produkt wurde abfiltriert, mit Wasser gewaschen und bei 50°C/20 Torr getrocknet. Man erhielt 4,36 g Produkt, was einer Ausbeute von 77% entsprach.

### c) Herstellung von 2,4-Diamino-6-piperidinyl-pyrimidin-3-N-oxid (Minoxidil

2,0 g 6-Chlor-2,4-diaminopyrimidin-3-N-oxid (0,0125 Mol) und 20,0 g (0,23 Mol) Piperidin wurden bei 101°C während 2 Stunden gerührt. Das Reaktionsgemisch wurde auf Raumtemperatur gekühlt. Der Festkörper wurde abfiltriert, mit Wasser gewaschen und bei 50°C/20 Torr getrocknet. Man erhielt 2,15 g weisses Pulver, Smp. über 258°C, Ausbeute 83%. Das Produkt war spektroskopisch identisch mit authentischem Minoxidil.
- ¹H-NMR (DMSO-d6): 1,52 (m, 6H)
3,39 (t, 4H)
5,39 (s, 1H)
6,72 (bs, 4H)
- IR (KBr) cm⁻¹: 3450, 3422, 3400, 3373, 3273, 1644, 1250, 1211, 1158, 1021

### Beispiel 2

### a) 2,4-Diamino-6-hydroxypyrimidin-3-oxid Hydrat

10,4 g Hydroxylaminhydrochlorid (0,15 Mol), 4,2 g Cyanamid (0,1 Mol) und 20 g Methanol wurden bei 20 bis 25°C zusammengegeben. Anschliessend wurden während 15 Minuten 27 g Natriummethylat (30% in Methanol, 0,15 Mol) zudosiert. Nach 45 Minuten wurde der Natriumniederschlag abfiltriert. Das Filtrat gab man zu einem Gemisch von 17,82 g Cyanessigsäuremethylester (0,18 Mol) und 32,4 g Natriummethylat (30% in Methanol, 0,18 Mol). Das Reaktionsgemisch wurde während 4 Stunden bei 22°C und anschliessend während 2 Stunden bei Rückflusstemperatur gerührt. Bei 55 bis 60°C wurde mit 18%iger Salzsäurelösung auf pH 4,5 gestellt. Das Methanol wurde dann unter Vakuum entfernt. Nach Abkühlen auf 10 bis 15°C wurde der Festkörper abfiltriert, mit Wasser gewaschen und bei 50°C/20 Torr getrocknet. Man erhielt 10,16 g des Titelproduktes. Die Ausbeute betrug 65,3% bezogen auf Cyanamid. Smp. über 300°C.
- IR (KBr) cm⁻¹: 3401, 3325, 3218, 3200, 3156, 1708, 1650, 1553, 1515, 1488, 1446, 1256, 1146, 992.
- UV (EtOH)nm:: 224, 277.

| Elementaranalyse | | | |
|---|---|---|---|
| ber. | C 30,0% | H 5,0% | N 35,0% (Hydrat) |
| gef. | C 30,2% | H 5,1% | N 34,9% |

### b) 2,4-Diamino-6-chlorpyrimidin-3-oxid Hydrochlorid

170,0 g 2,4-Diamino-6-hydroxy-3-oxid Hydrat (1,1 Mol), 1283 g Phosphoroxychlorid (8,4 Mol) wurden zusammengegeben. 215 g N,N′-Dimethylanilin (1,8 Mol) wurden bei 72°C während 30 Minuten zudosiert. Das Reaktionsgemisch wurde dann bei Rückflusstemperatur während 15 Stunden gerührt. Das überschüssige Phosphoroxychlorid wurde unter Vakuum bei 80°C abdestilliert. Den Rückstand gab man während 20 Minuten zu 1400 g Wasser von 20°C. Die entstandene Suspension wurde während 2 Stunden bei 5°C gerührt und filtriert. Das Produkt wurde mit kaltem Wasser gewaschen und bei 50°C/20 Torr getrocknet. Man erhielt 177,3 g des Titelproduktes, entsprechend einer Ausbeute von 80%. Smp. 142°C (zers.)
- ¹H-NMR (DMSO-d6): 8,05 (bs, 4H)
6,17 (s, 1H)
HCl kommt mit Wasserpeak zusammen
- IR (KBr) cm⁻¹: 3374, 3296, 3210, 3171, 3094, 1677, 1627, 1567, 1375, 1296, 1205, 945.
- UV (H20) nm:: 228, 290

### Beispiel 3

### Herstellung von 2,4-Diamino-6-hydroxypyrimidin-3-oxid Hydrat

11,5 g Chlorformamidiniumhydrochlorid (0,1 Mol), hergestellt nach der Vorschrift der DE-OS 19 15 668, wurden in 20 g Methanol gelöst. Dann wurden 18 g Natriummethylat (30% in Methanol, 0,1 Mol) bei 15 bis 20°C zugegeben. Eine Lösung von 3,3 g Hydroxylamin in 20 g Methanol wurde bei 15°C zugegeben und das Reaktionsgemisch bei 15 bis 20°C während 3 Stunden gerührt. 10 g Cyanessigsäuremethylester (0,1 Mol) und nachher 18 g Natriummethylat (30% in Methanol, 0,1 Mol) wurden zugegeben und während 2 Stunden bei Rückflusstemperatur gehalten. Das Gemisch wurde bei 55 bis 60°C mit 18%iger Salzsäurelösung auf pH 4,5 gestellt. Dann wurde soviel Methanol unter Vakuum abgedampft, dass die Rührbarkeit des Gemisches gewährleistet blieb. Nach Abkühlen auf 10 bis 15°C und 10 Minuten Rühren, wurde der Feststoff abfiltriert. Der Feststoff wurde mit Wasser gewaschen und bei 50°C/20 Torr getrocknet. Man erhielt 6,4 g beiges Pulver mit einem Gehalt von 47,6% (Rohprodukt). Die Ausbeute betrug 21% bezogen auf Chlorformamidiniumhydrochlorid.
Das Produkt war analytisch identisch mit dem Produkt aus Beispiel 2a).

## Patentansprüche

1. Verfahren zur Herstellung von 2,4-Diamino-6-piperidinylpyrimidin-3-N-oxid, dadurch gekennzeichnet, dass man Hydroxyguanidin mit einem Cyanessigsäurester unter Ringbildung zum 2,4-Diamino-6-hydroxypyrimidin-3-N-oxid überführt, das N-Oxid durch Chlorierung mit POCl₃ in Gegenwart eines Amins als Katalysator zum 2,4-Diamino-6-chlorpyrimidin-3-N-oxid chloriert und dieses mit Piperidin zum gewünschten Endprodukt umsetzt.

2. Verfahren nach Patentanspruch 1, dadurch gekennzeichnet, dass das Hydroxyguanidin hergestellt worden ist, indem man Hydroxylamin und Cyanamid in einem niedrigen Alkohol als Lösungsmittel in Gegenwart eines Alkalialkoholats in situ umsetzt.

3. Verfahren nach Patentanspruch 1, dadurch gekennzeichnet, dass das Hydroxyguanidin hergestellt worden ist, indem man eine wässrige Cyanamidlösung mit einer wässrigen Halogenwasserstoffsäure zum entsprechenden Halogenformamidiumhalogenid umsetzt und dieses mit Hydkroxylamin in Gegenwart eines Alkalialkoholats in einem niederen Alkohol umsetzt.

4. Verfahren nach Patentanspruch 3, dadurch gekennzeichnet, dass als wässrige Halogenwasserstoffsäure Salzsäure verwendet wird.

5. Verfahren nach Patentansprüchen 1 bis 4, dadurch gekennzeichnet, dass man das Hydroxylamin als Hydrochlorid anwendet.

6. Verfahren nach Patentansprüchen 1 bis 5, dadurch gekennzeichnet, dass man als niedrigen Alkohol Methanol und als Alkalialkoholat Natriummethylat verwendet.

7. Verfahren nach Patentansprüchen 1 bis 6, dadurch gekennzeichnet, dass tertiäre Amine, bevorzugt das N,N-Dimethylanilin, als Katalysator für die Umsetzung mit POCl₃ eingesetzt werden.

8. Verfahren nach Patentansprüchen 1 bis 7, dadurch gekennzeichnet, dass man das 2,4-Diamino-6-hydroxypyrimidin-3-N-oxid isoliert.

9. Verfahren nach Patentansprüchen 1 bis 8, dadurch gekennzeichnet, dass man das 2,4-Diamino-6-chlorpyrimidin-3-N-oxid isoliert.

10. 2,4-Diamino-6-hydroxypyrimidin-3-N-oxid.

## Claims

1. A process for the preparation of 2,4-diamino-6-piperidinyl pyrimidine-3-N-oxide, characterized by converting hydroxyguanidine with a cyanoacetic acid ester under ring formation into 2,4-diamino-6-hydroxypyrimidine-3-N-oxide, chlorinating the N-oxide with POCl₃ in the presence of an amine as catalyst to 2,4-diamino-6-chloropyrimidine-3-N-oxide, and reacting the latter with piperidine to the desired product.

2. The process according to Claim 1, characterized in that the hydroxyguanidine has been prepared through in-situ reaction of hydroxylamine and cyanamide in a lower alcohol as solvent and in the presence of an alkali alcoholate.

3. The process according to Claim 1, characterized in that the hydroxyguanidine has been prepared by reacting an aqueous cyanamide solution with an aqueous hydrogen halide acid to form the respective halogen formamidium halide and subsequently reacting the latter with hydroxyl amine in the presence of an alkali alcoholate in a lower alcohol.

4. The process according to Claim 3, characterized by using hydrochloric acid as an aqueous hydrogen halide acid.

5. The process according to Claims 1 to 4, characterized by using hydroxyl amine in the form of its hydrochloride.

6. The process according to Claims 1 to 5, characterized by using methanol as a lower alcohol and sodium methylate as an alkali alcoholate.

7. The process according to Claims 1 to 6, characterized by using tertiary amines, preferably N,N-dimethyl aniline, as a catalyst for the reaction with POCl₃.

8. The process according to Claims 1 to 7, characterized in that the 2,4-diamino-6-hydroxypyrimidine-3-N-oxide is isolated.

9. The process according to Claims 1 to 8, characterized in that the 2,4-diamino-6-chloropyrimidine-3-N-oxide is isolated.

10. 2,4-Diamino-6-hydroxypyrimidine-3-N-oxide.

## Revendications

1. Procédé pour la préparation de 2,4-diamino-6-pipéridinylpyrimidine-3-N-oxyde, caractérisé en ce que l'on réduit l'hydroxyguanidine avec un ester de l'acide cyanacétique avec cyclisation en 2,4 diamino-6-hydroxypyrimidine-3-N-oxyde, en ce que l'on chlore le N-oxyde par chloruration avec POCL₃ en présence d'une amine comme catalyseur en 2-4-diamino-6-chlorpyrimidin-3-N-oxyde et que l'on transforme celui-ci avec la pipéridine en le produit final souhaité.

2. Procédé selon la revendication 1, caractérisé en ce que l'hydroxyguanidine a été préparée en faisant réagir in situ l'hydroxylamine et le cyanamide avec un alcool inférieur comme solvant en présence d'un alcoolat alcalin.

3. Procédé selon la revendication 1, caractérisé en ce que l'hydroxyguanidine a été préparée en mettant en réaction une solution aqueuse de cyanamide avec un hydracide halogéné aqueux pour donner l'haloformamidiumhalogénure correspondant et en faisant réagir celui-ci avec l'hydroxylamine en présence d'un alcoolat alcalin dans un alcool inférieur.

4. Procédé selon la revendication 3, caractérisé en ce qu'en tant qu'hydracidehalogéné aqueux on utilise de l'acide chlorhydrique.

5. Procédé selon les revendications 1 à 4, caractérisé en ce que l'on utilise l'hydroxylamine en tant que chlorhydrate.

6. Procédé selon les revendications 1 à 5, caractérisé en ce qu'en tant qu'alcool inférieur on utilise le méthanol et en tant qu'alcoolat alcalin on utilise le méthylat de sodium.

7. Procédé selon les revendications 1 à 6, caractérisé en ce que l'on met en oeuvre en tant que catalyseur l'amine tertiaire, de préférence la N,N-diméthylaniline, pour la réaction avec POCL₃.

8. Procédé selon les revendications 1 à 7, caractérisé en ce que l'on isole le 2-4-diamino-6-hydroxypyrimidine-3-N-oxyde.

9. Procédé selon les revendications 1 à 8, caractérisé en ce que l'on isole de 2-4-diamino-6-chloropyrimidine-3-N-oxyde.

10. 2,4-diamino-6-hydroxypyrimidine-3-N-oxyde.
